# EUROPEAN PATENT SPECIFICATION

(11) **EP 1 732 620 B1**
(45) Date of publication and mention of the grant of the patent: **23.07.2008**
(21) Application number: 05726035.8
(22) Date of filing: 22.03.2005
(51) Int. Cl.: A61L 31/04

(54) **NEW FREE-RADICAL SCAVENGER CONTAINING VISCOELASTIC COMPOSITION, METHODS OF USE AND PACKAGE**
NEUER RADIKALFÄNGER MIT EINER VISKOELASTISCHEN ZUSAMMENSETZUNG; HERSTELLUNGSVERFAHREN UND VERPACKUNG
NOUVEAU PHAGOCYTE DE RADICAL LIBRE CONTENANT UNE COMPOSITION VISCOELASTIQUE, METHODES D'UTILISATION ET CONDITIONNEMENT

(30) Priority: 29.03.2004 US 812551
(43) Date of publication of application: 20.12.2006
(73) Proprietor: BAUSCH & LOMB INCORPORATED, Rochester, New York 14604-2701 (US)
(72) Inventor: BUCOLO, Claudio, I-95020 Acicastello (Catania) (IT); CRO, Melina, G., Catania (IT); MALTESE, Adriana, L. A., I-95030 Tremestieri Etneo (Catania) (IT); JANI, Dharmendra, M., Fairport, NY 14450 (US)
(74) Representative: Maiwald, Walter
(86) International application number: PCT/US2005/009512
(87) International publication number: WO 2005/097226

(56) References cited:
- EP-A- 0 214 853
- EP-A- 0 464 727
- EP-A- 0 781 547
- WO-A-03/072081
- US-A- 5 631 243

## Description

### BACKGROUND OF THE INVENTION

### Field of the Invention

This invention relates to a viscoelastic composition, method of use and related device used in viscosurgical applications and more particularly to a viscoelastic composition used in ophthalmic surgical application such as cataract removal surgery.

### Discussion of the Related Art

In the past decade, advances in the technology of eye surgery have made surgical treatment of eye disease and deformities attractive to alternative therapies. Cataract removal is one of the more common surgical procedure. Cataracts are opacities of the ocular lens, which generally arise in the elderly. Typically, cataract surgery involves removal of the cataractous lens from the capsular bag and replacement of the cataractous lens with a synthetic intraocular lens. Presently, this procedure involves making an incision through the sclera into the anterior chamber of the patient's eye. Another incision is made into the capsular bag. The cataractous lens is fractured in the capsular bag by procedures such as phacoemulsification and removed from the capsular bag by procedures such as aspiration. Thereafter, an intraocular lens is inserted into the capsular bag and deployed therein.

The overall procedure is potentially traumatic to the capsular bag and the tissue surrounding the anterior chamber. It is advantageous to reduce the amount of trauma to any living tissue in the patient's eye during a surgical procedure. Particularly, lens endothelial cells in the capsular bag are sensitive to damage. Damage to the lens endothelial cells is often permanent. Serious damage can cause loss of eyesight and failure of the surgical procedure.

Moreover, the process of phacoemulsification produces free radicals and/or oxidants. Free radicals and/or oxidants are unstable and react somewhat indiscriminately with biological molecules in tissue. For example, a free radical and/or oxidant that are produced in phacoemulsification can damage proteins, cell walls or even the DNA of a cell. It is advantageous to reduce the damage caused by these free radicals and/or highly reactive ions.

Viscoelastic compositions are injected in the anterior chamber of the eye and the capsular bag during surgery to protect the tissue from physical trauma. The viscoelastic compositions provide a physical barrier or cushion between the instruments and the tissue. Furthermore, viscoelastic compositions maintain the shape of a cavity during operation including the anterior chamber and capsular bag. Viscoelastic compositions have been known to contain agents that are free radical scavengers and/or antioxidants.

Selection of an ingredient in a viscoelastic composition for the purpose of controlling free-radical activity and/or antioxidants, require satisfying several criteria. The ingredient cannot negatively impact the viscoelastic properties, irritate tissue or cause an adverse immune response. The ingredient should be effective as a free-radical scavenger and/or antioxidant under conditions of desirable pH and osmolality. Of course, the effectiveness of the free-radical scavenger to dampen free radical activity is an important factor.

U.S Patent No. 5,880,107 discloses a viscoelastic composition for use in eye surgery. The viscoelastic composition contains hyaluronic acid as the primary ingredient to provide appropriate viscoelasticity. The composition further contained a citric acid salt, typically tri-sodium citrate, an antioxidant tolerated by the intraocular tissues and a phosphate buffer. The antioxidant is selected from the group comprising glucose, sulphides, superoxide dismutase (SOD), cysteine and derivates thereof. Furthermore, other antioxidants that could be used include antioxidants, which have at least one -SH or -CHO group, peptides and enzymes.

US Patent No. 6,086597 discloses a sodium hyaluronate viscosurgical composition that contains a compound to as a scavenger including superoxidedismutase, mannitol and glutathione.

US Patent No. 5,631,243 discloses a collagen-based viscosurgical composition. The composition has higher solubility at pH values close to neutral pH. Osmolarity is increased using nonionic solutes including glycerol, sorbitol, xylitol, threitol, mannitol, etc.

Tris[hydroxymethyl]-aminomethane is a quaternary ammonium compound that is found as an ingredient in a buffer system in topical ophthalmic formulations. See US Publ. No. 2003-0232089 and WO03/072081.

While significant improvements have been made in the rheological properties of viscoelastic compositions, there still exists a need for a composition that reduces the free radical and/or oxidant quenching activity without negatively impacting the viscoelastic properties of the viscoelastic composition. The present invention addresses these and other needs.

### SUMMARY OF THE INVENTION

The present invention is directed to a viscoelastic composition comprising an aqueous solution having a minimum of about 0.01%w/v and a maximum of about 20%w/v of a viscoelastic polymer based upon the total volume of the viscoelastic composition. Typically, the viscoelastic composition further contains tris[hydroxymethyl]aminomethane. Preferably, the viscoelastic composition and viscoelastic polymer is viscosurgically pure.

In one embodiment, the viscoelastic composition further comprises a polyol, including pentahydric alcohols, hexahydric alcohols and heptahydric alcohols and mixtures thereof. In one embodiment, the polyol is mannitol or sorbitol or mixtures thereof.

In another embodiment, there is a package for a viscoelastic composition, the package comprising a syringe containing a viscoelastic composition according to any embodiment, aspect, feature, combination or concept disclosed herein.

### DETAILED DESCRIPTION OF THE INVENTION

### Introduction

The present invention is directed to a viscoelastic composition comprising an aqueous solution having a minimum of about 0.01%w/v and a maximum of about 20%w/v of a viscoelastic polymer based upon the total volume of the viscoelastic composition. Typically the viscoelastic composition further contains tris[hydroxymethyl]aminomethane. The present invention also includes methods of use and a device.

### Definitions

Viscosurgically pure as it pertains to a viscoelastic composition or ingredient thereof is defined as a level of purity that is sufficiently free of impurities to meet or exceed the United States Food and Drug Administration standards for a viscosurgical viscoelastic effective at the time this application is filed.

Polysaccharides are defined as saccharides that have 10 or more saccharide monomer units.

Zero-shear viscosity is defined as the extrapolation of the viscosity of a liquid to a zero-shear rate from measurements of viscosity as the shear rate approaches zero measured on a plate and cone rheometer at 34°C.

High-shear viscosity is defined as the viscosity of a liquid measured on a plate and cone rheometer at 34 °C with a shear rate of 300 s⁻¹.

Pseudoplastic material is defined as a material that has relatively high viscosity under low-shear and relatively low viscosity under high-shear conditions.

The phrase "removing substantially all", as it relates to lenses and lens fragments, is defined as removing a sufficient quantity that an effective implantation of an intraocular lens is not inhibited thereafter. According to one embodiment, an effective removal of the lens requires a minimum of 90%w/v of the lens, 95%w/v of the lens or 98%w/v of the lens.

A cannula is defined as any tubular member having a passage that is configured to penetrate tissue and deliver a device through the passage.

A polyol for the purpose of this application is defined as a hydrocarbon having a hydroxyl group attached to each of the carbon atoms of the hydrocarbon.

A pentahydric alcohol is defined as a linear polyol having five carbon atoms.

A hexahydric alcohol is defined as a linear polyol having six carbon atoms.

A heptahydric alcohol is defined as a linear polyol having seven carbon atoms.

The percentage of quenching as describe in the application with the exception of the examples is defmed as the percentage amount that free-radical activity is prevented as evaluated by the 2-deoxy-D-ribose (2-DR) oxidation method. This is a conventional method of OH-radical detection forming by the Fenton reaction, radiation or ultrasound. It is based on its reaction with 2-DR. The obtained product of degradation, after a thermoactivated reaction with thiobarbituric acid (TBA), produces a pink chromogen quantified by HPLC.

### Formulation

According to one embodiment of the present invention, there is a viscoelastic composition comprising an aqueous solution having a minimum of about 0.01%w/v and a maximum of about 20%w/v of a viscoelastic polymer based upon the total volume of the viscoelastic composition. Typically the viscoelastic composition further contains tris[hydroxymethyl]aminomethane.

In one embodiment, the viscoelastic composition has a concentration of tris[hydroxymethyl]aminomethane that is a maximum of about 50mM and a minimum of about 0.1mM based upon the total weight of the viscoelastic composition. Typically, the concentration of tris[hydroxymethyl]aminomethane is a maximum of about 30mM and a minimum of about 0.5mM based upon the total volume of the viscoelastic composition. Preferably, the concentration of tris[hydroxymethyl]aminomethane is a minimum of about 0.5mM, about 0.7mM or about 0.9mM and/or a maximum of about 15mM, about 20mM or about 25mM based upon the volume of the viscoelastic composition in one aspect of the invention.

In one embodiment, the viscoelastic composition also contains a polyol. The polyol in the viscoelastic composition, optionally, is selected from the group comprising pentahydric alcohols, hexahydric alcohols and heptahydric alcohols and mixtures thereof. Preferably, the polyol is a hexahydric alcohol. More preferably, the polyol is xyletol, mannitol and/or sorbitol.

The viscoelastic composition has a concentration of the polyol, including pentahydric alcohols, hexahydric alcohols and heptahydric alcohols and mixtures thereof, that is a minimum of about 0.1%w/v and/or a maximum of about 15%w/v based upon the total volume of the viscoelastic composition. Typically, the concentration of the polyol including pentahydric alcohols, hexahydric alcohols and heptahydric alcohols and mixtures thereof, is a minimum of about 0.3%w/v, about 0.5%w/v or about 1%w/v and/or a maximum of about 10%w/v, about 6%w/v or about 4%w/v based upon the total volume of the viscoelastic composition. Optionally, the concentration of xyletol, mannitol and or sorbitol is at one or more concentrations in the range disclosed above for polyols.

The viscoelastic composition of one embodiment of the present invention has a ratio of the viscosity of the viscoelastic composition to the viscosity of a comparable viscoelastic composition having no polyol and tris[hydroxymethyl]aminomethane that is a minimum of about 1 and a maximum of about 2.5. A comparable viscoelastic composition is defined as a viscoelastic composition that has all of the same chemical ingredients as the viscoelastic composition at the same concentrations except it has no polyol and tris[hydroxymethyl]aminomethane. Typically, the ratio of the viscosity of the viscoelastic composition to the viscosity of a comparable viscoelastic composition is a minimum of about 1, about 1.1 and about 1.2 and a maximum of about 2.5, about 2.2 and about 2.

The viscoelastic composition of yet another embodiment quenches chemical scavengers effectively, wherein the percentage of quenching is a minimum of about 75%. Typically, the percentage quenching is greater than about 80%, about 85%, about 87%, about 90% or about 92% according to the method of testing in Example 9 herein.

The viscoelastic composition comprises one or more viscoelastic polymers that are useful and known as viscosurgical devices. In one embodiment, the viscoelastic polymer is selected from the group comprising hyaluronic acid, hydroxypropylmethylcellulose, polyacrilyc acid, carbopol, polyvinylalchol, polyvinylpirrolidone, condroitin sulfate, polycarbophil, methylcellulose, carboxymethylcellulose, hydroxyethylcellulose, hydroxypropylcellulose, ethylcellulose, polyethylene oxides, alginate, pectin, xanthan gum, dextrans, collagen and derivatives threof and salts thereof and combinations thereof.

In one embodiment, the average molecular weight of the viscoelastic polymer, including a polysaccharide, is a minimum of about 20 kD and a maximum of about 5,000 kD. Generally, the average molecular weight of a viscoelastic polymer, including polysaccharide, is a minimum of about 30 kD, about 50 kD, about 70 kD, about 400 kD, about 500 kD, about 750 kD or about 1,000 kD. Typically, the average molecular weight of a viscoelastic polymer, including a polysaccharide, is a maximum of about 50 kD, about 80 kD, about 100 kD, about 200 kD, about 400 kD, about 500 kD, about 1,000 kD or about 3,000 kD.

Typically, there are two general classes of viscoelastic compositions. A dispersive viscoelastic composition has properties that disperse or coat the tissue well and adhere well to the tissue. A dispersive viscoelastic composition (also known as an "adhesive viscoelastic composition") typically has a low molecular weight. A cohesive viscoelastic composition is better at maintaining the space in a cavity in human tissue and is less likely to leak from the cavity under low or zero shear conditions. Typically, a cohesive viscoelastic composition has a high molecular weight.

In one embodiment, the average molecular weight of a viscoelastic polymer in a dispersive viscoelastic composition is a minimum of about 20 kD, 30 kD, about 50 kD or about 70 kD. Typically, the average molecular weight of a viscoelastic polymer in a dispersive viscoelastic composition is a maximum of about 50 kD, about 80 kD, about 100 kD, about 200 kD, about 400 kD or about 500 kD.

In another embodiment, the average molecular weight of a viscoelastic polymer in a cohesive viscoelastic composition is a minimum of about 400 kD, about 500 kD, about 750 kD or about 1,000 kD. Typically, the average molecular weight of a viscoelastic polymer in a cohesive viscoelastic composition is a maximum of about 1,000 kD, 3,000 kD or about 5,000 kD.

The concentration of the viscoelastic polymer is a minimum amount of about 0.01%w/v and a maximum amount of about 20%w/v based upon the total weight of the viscoelastic composition in one embodiment. Typically, the concentration of the viscoelastic polymer is a minimum of about 0.1%w/v, about 0.2%w/v, about 1.0 or about 2.0%w/v and a maximum of about 0.3%w/v, about 0.5%w/v, about 1%w/v, about 2%w/v, about 3%w/v, about 5%w/v or about 15%w/v based upon the total weight of the viscoelastic composition.

In still another embodiment, the viscoelastic polymer comprises a mixture of hyaluronic acid and/or salts thereof and hydroxypropylmethylcellulose.

The concentration of hyaluronic acid and/or salts thereof is a minimum of about 0.1%w/v and a maximum of about 6%w/v based upon the volume of the viscoelastic composition in one embodiment. Typically, the concentration of hyaluronic acid and/or salts thereof is a minimum of about 0.3%w/v, about 0.6%w/v or about 1%w/v and/or a maximum of about 6%w/v, about 4%w/v or about 2%w/v based upon the volume of the viscoelastic composition.

The average molecular weight of the hyaluronic acid and/or salts thereof is a minimum of about 500 kD and/or a maximum of about 5000 kD in one embodiment. Typically, the average molecular weight of the hyaluronic acid and/or salts thereof is a minimum of about 500 kD, about 700kD or about 1000kD and/or a maximum of about 4000kD, about 3000kD or about 2000kD.

The concentration of hydroxypropylmethylcellulose is a minimum of about 0.05%w/v and/or a maximum of about 5%w/v based upon the volume of the viscoelastic composition in one embodiment. Typically, the concentration of hydroxypropylmethylcellulose is a minimum of about 0.2%w/v, about 0.4%w/v or about 0.8%w/v and/or a maximum of about 5%w/v, about 3%w/v or about 1%w/v based upon the volume of the viscoelastic composition.

The average molecular weight of the hydroxypropylmethylcellulose is a minimum of about 10 kD and/or a maximum of about 120 kD according to one embodiment. Typically, the average molecular weight of the hydroxypropylmethylcellulose is minimum of about 10 kD, about 12 kD or about 20 kD and/or a maximum of about 120 kD, about 90 kD or about 86 kD.

In one embodiment, the viscoelastic polymer comprises a polysaccharide. In another embodiment, the viscoelastic polymer is preferably a polysaccharide selected from the group comprising hyaluronic acid, hydroxypropylmethylcellulose, condroitin sulfate, methylcellulose, carboxymethylcellulose, hydroxyethylcellulose, hydroxypropylcellulose, ethylcellulose, alginate, pectin, dextrans, collagen, proteoglycans, keratin carrageenans and derivatives thereof and salts thereof and combinations thereof.

The viscoelastic polymer comprises alginate in one embodiment. Typically the concentration of alginate is a minimum of about 0.05%w/v and/or a maximum of about 9%w/v based upon the volume of the viscoelastic composition. Optionally, the minimum alginate concentration is about 1%w/v, about 1.5%w/v, about 2%w/v, about 3%w/v or about 4%w/v based upon the total weight of the viscoelastic composition. Optionally, the maximum alginate concentration is about 10%w/v, about 8%w/v, about 6%w/v, about 4%w/v, about 3%w/v or about 2%w/v based upon the total weight of the viscoelastic composition. Preferably; the alginate concentration is a minimum of about 2%w/v and/or a maximum of about 5.25%w/v.

In one embodiment, the average molecular weight of the alginate is a minimum of about 50 kD and/or a maximum of about 5,000 kD. Typically, the average molecular weight of the alginate is a minimum of about 100 kD, about 200 kD, about 500 kD or about 1000 kD. Typically, the average molecular weight of the alginate is a maximum of about 2000 kD, about 1000 kD, about 750 kD or about 500 kD.

The viscoelastic composition has one or more properties including osmolality, pH, zero-shear viscosity and high-shear viscosity. The osmolality of the viscoelastic composition is a minimum of about 200mOsmol/Kg and a maximum of about 400mOsmol/Kg in an embodiment. Typically, the osmolality of the viscoelastic composition is a minimum of about 220mOsmol/Kg, about 260mOsmol/Kg, about 280mOsmol/Kg, about 300mOsmol/Kg or about 320mOsmol/Kg and a maximum of about 400mOsmol/Kg, about 380mOsmol/Kg, about 360mOsmol/Kg or about 340mOsmol/Kg.

The zero-shear viscosity of the viscoelastic composition is a minimum of about 6.10⁴ cps and a maximum of about 4.10⁶ cps. Generally, the zero-shear viscosity of the viscoelastic composition is a minimum of about 6x10⁴ cps, about 4x10⁵ cps or about 8x10⁵ cps and/or a maximum of about 3.5x10⁶ cps, about 1.8x10⁶ cps or about 1.2x10⁶ cps.

The high-shear viscosity of the viscoelastic composition is a minimum of about 500 cps and/or a maximum of about 2000 cps. Generally, the high-shear viscosity of the viscoelastic composition is a minimum of about 500 cps, about 600 cps or about 700 cps and/or a maximum of about 2000 cps, about 1500 cps or about 1000 cps.

The pH of the viscoelastic composition of one embodiment is a minimum of about 5 and a maximum of about 8. In one embodiment, the pH of the viscoelastic composition is a minimum of about 5.5, about 6 or about 6.5 and/or a maximum of about 7.5, about 7.2 or about 7.

The viscoelastic composition of one embodiment has a formulation set forth in Table 1.

| TABLE 1 | |
|---|---|
| **Component or Property of the Viscoelastic Composition** | **Amount** |
| 1.0x10⁶-3x10⁶ Molecular Weight Hyaluronic Acid or Salt Form Thereof | 0.5%w/v to 3%w/v |
| 20,000-200,000 Molecular Weight Hydroxypropylmethylcellulose | 0.1%w/v to 2%w/v |
| Sorbitol | 0.1%w/v to 20%w/v |
| Tris[hydroxymethyl]aminomethane | 1mM to 100mM |
| Buffered to pH | 6.9 to 7.5 |
| Osmolality adjusted to | 290-350 mOsm/Kg |

In one preferred embodiment, the viscoelastic composition comprises the following:
2.3%w/v hyaluronic acid (MW 1.98x10⁶)
0.8%w/v hydroxypropylmethylcellulose (MW 86,000)
4.4%w/v sorbitol
20 mM tris[hydroxymethyl]aminomethane
purified water q. s. to 100 ml
pH 7.3
335 mOsm/Kg

In another preferred embodiment, the viscoelastic composition comprises the following:
2%w/v hyaluronic acid (MW 1.98x10⁶)
0.8%w/v hydroxypropylmethylcellulose (MW 86,000)
4.4%w/v sorbitol
20 mM tris[hydroxymethyl] aminomethane
purified water q. s. to 100 ml
pH 7.3
335 mOsm/Kg

In another preferred embodiment, the viscoelastic composition comprises the following:
2 %w/v hyaluronic acid (MW 1.98x10⁶)
1 %w/v hydroxypropylmethylcellulose (MW 86,000)
4.4 %w/v sorbitol
20 mM tris[hydroxymethyl] aminomethane
purified water q. s. to 100 ml
pH 7.3
335 mOsm/Kg

In another preferred embodiment, the viscoelastic composition comprises the following:
5.25 %w/v alginate
4.4 %w/v sorbitol
20 mM tris[hydroxymethyl]aminomethane
Purified water q. s. a. d. to 100 %w/v

In another preferred embodiment, the viscoelastic composition comprises the following:
1.4 %w/v hyaluronic acid
0.6 %w/v hydroxypropylmethylcellulose.
4.4. %w/v sorbitol
20 mM tris[hydroxymethyl]aminomethane
pH 7.3
Purified water q. s. a. d. to 100 %w/v

Viscoelastic composition according to any one or more of the foregoing embodiments, concepts or aspects including combinations and variations of the foregoing embodiments can be used as described in the following.

The viscoelastic composition can be used, e.g., in a method of maintaining space in a cavity in human tissue. The method comprises the step of injecting, into the cavity, a viscoelastic composition according to any embodiment, aspect, feature, combination or concept disclosed herein. Thereafter, the viscoelastic composition is removed from the cavity. Preferably, the cavity is the anterior chamber of the eye or the capsular bag.

The viscoelastic composition can also be used, e.g., in a method of protecting tissue from trauma during a surgical procedure. The method comprises the step of coating at least a portion of the tissue with a viscoelastic composition according to any embodiment, aspect, feature, combination or concept disclosed herein. Preferably, the tissue that is covered is in the anterior chamber of the eye and/or the capsular bag. A surgical procedure is then performed near the tissue. When the surgical procedure is completed, at least a portion of the viscoelastic composition is removed from the tissue.

The viscoelastic composition can also be used, e.g., in a method of replacing a natural lens from an eye. Examples of procedures for removing a lens from a patient's eye include but are not limited to U.S. Patent Nos. 3,589,363 (cataract surgery), 3,693,613 (phacoemulsification) and 5,718,676 (process using micro flow needle), which are all incorporated herein by reference in their entirety. The process generally includes providing a passage through a sclera or cornea into an anterior chamber of the eye. The process involves making a small incision into the sclera or cornea. Alternatively or additionally, a cannula or trochar is used to create a passage through the sclera or cornea. Preferably, the incision or passage is as small as possible. Preferably, the incision or passage is smaller than about 5 mm, about 4 mm or about 3mm. Thereafter, the aqueous humor is withdrawn or otherwise removed from the anterior chamber of the eye.

A viscoelastic composition can be also, e.g., inserted into the anterior chamber. The viscoelastic composition maintains the space in the anterior chamber. The viscoelastic composition coats the tissue in the wall of the anterior chamber.

According to one embodiment, there is a package for a viscoelastic composition that includes a delivery device. The device delivers a viscoelastic composition into the anterior chamber of a patient's eye. The device includes a syringe that contains a viscoelastic composition according to any embodiment, aspect, combination, concept or feature disclosed herein.

The syringe further comprises an outlet port and, optionally, a cannula configured to sealably connect to the outlet port. The cannula has a maximum inner diameter of about 2 mm. Typically, the maximum inner diameter is about 1.8 mm, about 1.5 mm or about 1 mm. Generally, the minimum inner diameter is about 0.8 mm, about 0.6 mm or about 0.4 mm.

In one embodiment, the viscoelastic composition requires a maximum force of 30 N to pass through a stainless steel cannula having a length of 2.2 cm and an inner diameter of 0.5 mm at a delivery rate of 0.02 ml/sec. Preferably, the viscoelastic composition requires a maximum force of about 27 N, about 25 N, about 20 N or about 18 N to pass through a stainless steel cannula having a length of 2.2 cm and an inner diameter of 0.5 mm at a delivery rate of 0.02 ml/sec.

Once the viscoelastic composition is inserted into the anterior chamber the corneal lens is removed. The technique for removing the lens includes performing a capsulorhexis incision and breaking down the lens into smaller pieces through phacoemulsification or other known techniques. Thereafter, the pieces are removed by, for example, aspiration.

The viscoelastic composition is inserted into the capsular bag for space maintenance purposes. Moreover, the viscoelastic composition coats the capsular bag and protects it for additional steps in the surgical procedure.

The intraocular lens can be also, e.g., inserted into the capsular bag. Typically, there is a method of inserting an intraocular lens into a capsular bag of an eye. The method comprises providing a lens insertion device comprising a loadable chamber configured to receive the intraocular lens, a tapered conduit having a first end connected to the loadable chamber and a second end. The second end is configured to penetrate through the passage in the corneal lens and into the capsular bag. An example of a lens insertion device is found in U.S. Patent No. 6,558,419, which is incorporated herein by reference in its entirety. The lens insertion device is further configured with a slidable actuator. The slidable actuator of one embodiment is configured to actuate the intraocular lens through the conduit past the second end. Typically, the second end of the tapered conduit has an inner diameter that is a maximum of about 5 mm. Preferably the second end of the tapered conduit has an inner diameter that is a maximum of about 4 mm about 3.5 mm, about 3 mm or about 2.8 mm. Preferably, a maximum force of about 30 N is required to deliver the intraocular lens through the cannula. More preferably, a maximum force of about 27 N, about 25 N, about 20 N or about 18 N is required to deliver the intraocular lens through the cannula.

Prior to deployment, at least a portion of the intraocular lens is coated with a viscoelastic composition according to any one of the embodiments, aspects, concepts, combinations or features of the present invention. The intraocular lens is loaded into the loadable chamber either before or after it is coated. The conduit is inserted through the passage. The actuator forces the intraocular lens through the passage and into the capsular bag. After the intraocular lens is deployed, the conduit is removed from the passage.

Typically, at least a portion of the viscoelastic composition is removed from the capsular bag and/or anterior chamber. A physiological solution is then used to fill the anterior chamber. The sclera and/or cornea are sutured to close the passage.

### EXAMPLES

### Example 1: Preparation of Formulation 1

The following mixture was prepared and labeled as Formulation 1:
2.3%w/v hyaluronic acid (MW 1.98x10⁶)
0.8%w/v hydroxypropylmethylcellulose (MW 86,000)
4.4%w/v sorbitol
20mM tris-[hydroxymethyl]aminomethane ("tris")
purified water q. s. to 100 ml
pH 7.3
335 mOsm/Kg

### Example 2: Preparation of Formulation 2

The following mixture was prepared and labeled as Formulation 2:
2%w/v hyaluronic acid (MW 1.98x10⁶)
0.8%w/v hydroxypropylmethylcellulose (MW 86,000)
4.4%w/v sorbitol
20mM tris
purified water q. s. a. d. to 100 ml
pH 7.3
335 mOsm/Kg

### Example 3: Preparation of Formulation 3

The following mixture was prepared and labeled as Formulation 3:
2%w/v hyaluronic acid (MW 1.98x10⁶)
1%w/v hydroxypropylmethylcellulose (MW 86,000)
4.4%w/v sorbitol
20 mM tris
purified water q. s. a. d. to 100 ml
pH 7.3
335 mOsm/Kg

### Example 4: Preparation of Formulation 4

The following formulation was prepared and labeled as Formulation 4:
2.3%w/v hyaluronic acid (MW 1.98x10⁶)
0.8%w/v hydroxypropylmethylcellulose (MW 86,000)
4.4%w/v sorbitol
Purified water q. s. a. d. to 100 ml
pH 7.3
335 mOsm/Kg

### Example 5: Preparation of Formulation 5

The following formulation was prepared and labeled as Formulation 5:
2.3%w/v hyaluronic acid (MW 1.98x10⁶)
0.8%w/v hydroxypropylmethylcellulose (MW 86,000)
Purified water q. s. a. d. to 100 ml
pH 7.3
335 mOsm/Kg

### Example 6: Preparation of Formulation 6

The following formulation was prepared and labeled as Formulation 6:
2.3%w/v hyaluronic acid (MW 1.98x10⁶)
0.8%w/v hydroxypropylmethylcellulose (MW 86,000)
20mM tris
Purified water q. s. a. d. to 100 ml
pH 7.3
335 mOsm/Kg

### Example 7: Preparation of Formulation 7

The following formulation was prepared and labeled as Formulation 7:
2.3%w/v hyaluronic acid (MW 1.98x10⁶)
4.4%w/v sorbitol
20 mM tris
Purified water q. s. a. d. to 100 ml
pH 7.3
335 mOsm/Kg

### Example 8: Preparation of Formulation 8

A commercial sample of Viscoat® was labeled as Preparation 8.

### Example 9: Free Radical Measurement of Formulations 1-8

The OH-scavenging activity of viscoelastic substances has been evaluated by the 2-deoxy-D-ribose (2-DR) oxidation method. This is a conventional method of OH-radical detection forming by the Fenton reaction, radiation or ultrasound. It is based on its reaction with 2-DR, which bring to the accumulation of 2-DR degradation products, especially malondialdehyde (MDA). The obtained product, after a thermo-activated reaction with thiobarbituric acid (TBA), produces a pink chromogen quantified by HPLC.

Stock solutions of 2-DR (40 mM), Fe²⁺/EDTA (10 mM), H₂O₂ (10mM in water (bubbled with N₂ for 30 min at room temperature) were prepared immediately before the experiment and stored on ice. An aliquot (500µl) of the formulations 1 through 8 and water (used as control) was added to 900 µl phosphate buffer solution (0.1M, pH 7.4) and shaken by vortex until the solution was homogeneous. Then 200µl of 2-DR, 200µl of 1mM Fe²⁺/EDTA and 200µl H₂O₂ were added and the solution was shaken by vortex for 1 min. The sample solutions were incubated for 1h at 37 °C and then added with 1ml TBA (2% in 0.1M phosphate buffer ph 7.4) and 1ml TCA (2% in 0.1M phosphate buffer pH 7.4). The samples were again incubated at 100 °C for 30 min and cooled in ice. 100µl of samples derived from Formulations 1 to 8, were diluted to 1ml volume with mobile phase and injected onto HPLC.

The processed Formulations 1 to 8 were chromatographed over a C18 column to detect the pink chromogen product (TBA-MDA complex) using an UV-VIS detector at 532 nm. Chromatograms for Formulations 1 through 8 were compared to the chromatogram for the comparative standard. The percentage of production of TBA-MDA complex in Formulations 1 through 8 was compared to the standard solution (control), calculated and shown in Table 2. No production of TBA-MDA complex correspond to one hundred percent quenching of free radical activity. The amount of TBA-MDA complex in the comparative standard (control) represents zero percent because no quenching of the free radical activity occurred. Each of the formulations containing tris[hydroxymethyl]aminomethane and/or sorbitol had higher free radical quenching than samples without either. Tris[hydroxymethyl]aminomethane and sorbitol individually have free-radical quenching properties. The combination of Tris[hydroxymethyl]aminomethane and sorbitol have the best free-radical quenching properties.

| Table 2: Percentage of Quenching of Free-Radical activity | | | | | |
|---|---|---|---|---|---|
| **Formu lations** | **%w/v HA (mw 1.98x10⁶)** | **%w/v HPMC (mw 8.6x10⁴)** | **%w/v Sorbitol** | **Tris (mM)** | **% of quenching** |
| 1 | 2.3 | 0.8 | 4.4 | 20 | 92 |
| 2 | 2 | 0.8 | 4.4 | 20 | 82 |
| 3 | 2 | 1.0 | 4.4 | 20 | 80 |
| 4 | 2.3 | 0.8 | 4.4 | - | 90 |
| 5 | 2.3 | 0.8 | - | - | 80 |
| 6 | 2.3 | 0.8 | - | 20 | 87.6 |
| 7 | 2.3 | - | 4.4 | 20 | 94 |
| 8 | - | - | - | - | 79 |
| Control | - | - | - | - | 0 |

## Claims

1. A viscoelastic composition comprising an aqueous solution having a minimum of about 0.01%w/v and a maximum of about 20%w/v of a viscoelastic polymer based upon the total volume of the viscoelastic composition and further having tris[hydroxymethyl]aminomethane.

2. The composition of claim 1, wherein the concentration of tris[hydroxymethyl]aminomethane is a maximum of about 50mM and a minimum of about 0.1mM based upon the total weight of the viscoelastic composition.

3. The composition of claim 1, further comprising a polyol.

4. The composition of claim 3, wherein at least one polyol is selected from the group comprising pentahydric alcohols, hexahydric alcohols and heptahydric alcohols and mixtures thereof.

5. The composition of claim 4, wherein the polyol is a hexahydric alcohol.

6. The composition of claim 5, wherein the polyol is mannitol.

7. The composition of claim 5, wherein the polyol is sorbitol.

8. The composition of claim 3, wherein the concentration of the polyol is a minimum of about 0.1%w/v and a maximum of about 15%w/v based upon the total volume of the viscoelastic composition.

9. The composition of claim 1, wherein the concentration of tris[hydroxymethyl]aminomethane is a minimum of about 0.5mM and a maximum of about 30mM.

10. The composition of claim 1, wherein the ratio of the viscosity of the viscoelastic composition to the viscosity of a comparable viscoelastic composition having no polyol and tris[hydroxymethyl]aminomethane is a minimum of about 1 and a maximum of about 2.5.

11. The composition of claim 1, wherein the percentage of quenching is a minimum of about 45%.

12. The composition of claim 1, wherein the viscoelastic polymer is selected from the group comprising hyaluronic acid, hydroxypropylmethylcellulose, polyacrilyc acid, carbopol, polyvinylalchol, polyvinylpirrolidone, condroitin sulfate, polycarbophil, methylcellulose, carboxymethylcellulose, hydroxyethylcellulose, hydroxypropylcellulose, ethylcellulose, polyethylene oxides, alginate, pectin, xanthan gum, dextrans, collagen and derivatives threof and salts thereof and combinations thereof.

13. The composition of claim 1, wherein the viscoelastic polymer comprises a polysaccharide.

14. The composition of claim 13, wherein the polysaccharide is selected from the group comprising hyaluronic acid, hydroxypropylmethylcellulose, condroitin sulfate, methylcellulose, carboxymethylcellulose, hydroxyethylcellulose, hydroxypropylcellulose, ethylcellulose, alginate, pectin, dextrans, collagen, proteoglycans, polyvinylpyrrolidone, keratin carrageenans and derivatives thereof and salts thereof and combinations thereof.

15. The composition of claim 1, wherein the viscoelastic polymer comprises alginate.

16. The composition of claim 15, wherein the concentration of alginate is a minimum of about 0.05%w/v and a maximum of about 9%w/v based upon the volume of the viscoelastic composition.

17. The composition of claim 15, wherein the average molecular weight of the alginate composition of yet minimum of about 50 kD and a maximum of about 5,000 kD.

18. The composition of claim 1, wherein the viscoelastic polymer comprises a mixture of hyaluronic acid and/or salts thereof and hydroxypropylmethylcellulose.

19. The composition of claim 18, wherein the concentration of hyaluronic acid and/or salts thereof is a minimum of about 0.1 %w/v and a maximum of about 6%w/v based upon the volume of the viscoelastic composition.

20. The composition of claim 19, wherein the average molecular weight of the hyaluronic acid and/or salts thereof composition of yet minimum of about 500 kD and a maximum of about 5000 kD.

21. The composition of claim 18, wherein the concentration of hydroxypropylmethylcellulose is a minimum of about 0.05%w/v and a maximum of about 5%w/v based upon the volume of the viscoelastic composition.

22. The composition of claim 21, wherein the average molecular weight of the hydroxypropylmethylcellulose composition of yet minimum of about 10 kD and a maximum of about 120 kD.

23. The composition of claim 1, wherein the average molecular weight of the viscoelastic polymer is a minimum of about 20 kD and a maximum of about 5,000 kD.

24. The composition of claim 1, wherein the osmolality of the viscoelastic composition is a minimum of about 200mOsmol/Kg and a maximum of about 400mOsmol/Kg.

25. The composition of claim 1, wherein the zero-shear viscosity of the viscoelastic composition is a minimum of about 6·10⁴ cps and a maximum of about 4.10⁶ cps.

26. The composition of claim 1, wherein the high-shear viscosity of the viscoelastic composition is a minimum of about 500 cps and a maximum of about 2000 cps.

27. The composition of claim 1, wherein the pH of the viscoelastic composition is a minimum of about 5 and a maximum of about 8.

28. A package for a viscoelastic composition, the package comprising a syringe containing the viscoelastic composition of claim 1.

29. The package of claim 28, wherein the syringe has an outlet port, the package further comprises a cannula configured to sealably connect to the outlet port having a maximum inner diameter of about 2 mm.

30. The package of claim 28, wherein viscoelastic composition requires a maximum force of 30 N to pass through the cannula.

## Patentansprüche

1. Eine viskoelastische Zusammensetzung umfassend eine wässrige Lösung, die mindestens etwa 0,01 w/v-% und höchstens etwa 20 w/v-% eines viskoelastischen Polymers basierend auf dem Gesamtvolumen der viskoelastischen Zusammensetzung enthält sowie zusätzlich Tris[hydroxymethyl]aminomethan.

2. Die Zusammensetzung gemäß Anspruch 1, wobei die Konzentration des Tris[hydroxymethyl]aminomethans höchstens etwa 50 mM und mindestens etwa 0,1 mM basierend auf dem Gesamtgewicht der viskoelastischen Zusammensetzung beträgt.

3. Die Zusammensetzung gemäß Anspruch 1, weiter umfassend ein Polyol.

4. Die Zusammensetzung gemäß Anspruch 3, wobei mindestens ein Polyol ausgewählt ist aus der Gruppe umfassend Pentahydroxyalkohole, Hexahydroxyalkohole und Heptahydroxyalkhole sowie Mischungen davon.

5. Die Zusammensetzung gemäß Anspruch 4, wobei das Polyol ein Hexahydroxyalkohol ist.

6. Die Zusammensetzung gemäß Anspruch 5, wobei das Polyol Mannitol ist.

7. Die Zusammensetzung gemäß Anspruch 5, wobei das Polyol Sorbitol ist.

8. Die Zusammensetzung gemäß Anspruch 3, wobei die Konzentration des Polyols mindestens etwa 0,1 w/v-% und höchstens etwa 15 w/v-% basierend auf dem Gesamtgewicht der viskoelastischen Zusammensetzung beträgt.

9. Die Zusammensetzung gemäß Anspruch 1, wobei die Konzentration des Tris[hydroxymethyl]aminomethans mindestens etwa 0,5 mM und höchstens etwa 30 mM beträgt.

10. Die Zusammensetzung gemäß Anspruch 1, wobei das Verhältnis der Viskosität der viskoelastischen Zusammensetzung zu der Viskosität einer vergleichbaren viskoelastischen Zusammensetzung ohne Polyol und Tris[hydroxymethyl]aminomethan mindestens etwa 1 und höchstens etwa 2,5 beträgt.

11. Die Zusammensetzung gemäß Anspruch 1, wobei der Prozentsatz des Quenchings mindestens etwa 45 % beträgt.

12. Die Zusammensetzung gemäß Anspruch 1, wobei das viskoelastische Polymer ausgewählt ist aus der Gruppe umfassend Hyaluronsäure, Hydroxypropylmethylcellulose, Polyacrylsäure, Carbopol, Polyvinylalkohol, Polyvinylpirrolidon, Chondroitinsulfat, Polycarbophil, Methylcellulose, Carboxymethylcelulose, Hydroxyethylcellulose, Hydroxypropylcellulose, Ethylcellulose, Polyethylenoxide, Alginat, Pektin, Xanthangummi, Dextrane, Collagen und Derivate davon sowie Salze davon und Kombinationen davon.

13. Die Zusammensetzung gemäß Anspruch 1, wobei das viskoelastische Polymer ein Polysaccharid umfasst.

14. Die Zusammensetzung gemäß Anspruch 13, wobei das Polysaccharid ausgewählt ist aus der Gruppe umfassend Hyaluronsäure, Hydroxypropylmethylcellulose, Chondroitinsulfat, Methylcellulose, Carboxymethylcellulose, Hydroxyethylcellulose, Hydroxypropylcellulose, Ethylcellulose, Alginat, Pektin, Dextrane, Collagen, Proteoglycane, Polyvinylpyrrolidon, Keratin-Carrageenane und Derivate davon sowie Salze davon und Kombinationen davon.

15. Die Zusammensetzung gemäß Anspruch 1, wobei das viskoelastische Polymer Alginate umfasst.

16. Die Zusammensetzung gemäß Anspruch 15, wobei die Konzentration des Alginates mindestens etwa 0,05 w/v-% und höchstens etwa 9 w/v-% basierend auf dem Volumen der viskoelastischen Zusammensetzung beträgt.

17. Die Zusammensetzung gemäß Anspruch 15, wobei das mittlere Molekulargewicht der besagten Alginatzusammensetzung mindestens etwa 50 kD und höchstens etwa 5000 kD beträgt.

18. Die Zusammensetzung gemäß Anspruch 1, wobei das viskoelastische Polymer eine Mischung von Hyaluronsäure und/oder Salzen davon und Hydroxypropylmethylcellulose umfasst.

19. Die Zusammensetzung gemäß Anspruch 18, wobei die Konzentration der Hyaluronsäure und/oder Salzen davon mindestens etwa 0,1 w/v-% und höchstens etwa 6 w/v-% basierend auf dem Volumen der viskoelastischen Zusammensetzung beträgt.

20. Die Zusammensetzung gemäß Anspruch 19, wobei das mittlere Molekulargewicht der Hyaluronsäure und/oder Salzen davon in der besagten Zusammensetzung mindestens etwa 500 kD und höchstens etwa 5000 kD beträgt.

21. Die Zusammensetzung gemäß Anspruch 18, wobei die Konzentration der Hydroxypropylmethylcellulose mindestens etwa 0,05 w/v-% und höchstens etwa 5 w/v-% basierend auf dem Volumen der viskoelastischen Zusammensetzung beträgt.

22. Die Zusammensetzung gemäß Anspruch 21, wobei das mittlere Molekulargewicht der besagten Hydroxypropylmethylcellulose-Zusammensetzung mindestens etwa 10 kD und höchstens etwa 120 kD beträgt.

23. Die Zusammensetzung gemäß Anspruch 1, wobei das mittlere Molekulargewicht des viskoelastischen Polymers mindestens etwa 20 kD und höchstens etwa 5000 kD beträgt.

24. Die Zusammensetzung gemäß Anspruch 1, wobei die Osmolarität der viskoelastischen Zusammensetzung mindestens etwa 200 mOsmol/kg und höchstens etwa 400 mOsmol/kg beträgt.

25. Die Zusammensetzung gemäß Anspruch 1, wobei die Null-Scherviskosität der viskoelastischen Zusammensetzung mindestens etwa 6·10⁴ cps und maximal etwa 4·10⁶ cps beträgt.

26. Die Zusammensetzung gemäß Anspruch 1, wobei die Hoch-Scherviskosität der viskoelastischen Zusammensetzung mindestens etwa 500 cps und höchstens etwa 2000 cps beträgt.

27. Die Zusammensetzung gemäß Anspruch 1, wobei der pH-Wert der viskoelastischen Zusammensetzung mindestens etwa 5 und höchstens etwa 8 beträgt.

28. Eine Verpackung für eine viskoelastische Zusammensetzung, wobei die Verpackung eine Spritze umfasst, welche die viskoelastische Zusammensetzung des Anspruchs 1 enthält.

29. Die Verpackung gemäß Anspruch 28, wobei die Spritze eine Auslassöffnung aufweist und die Verpackung zusätzlich eine Kanüle umfasst, die so gestaltet ist, um mit der Auslassöffnung, die einen maximalen Innendurchmesser von etwa 2 mm aufweist, verschließbar verbunden zu werden.

30. Die Verpackung gemäß Anspruch 28, wobei die viskoelastische Zusammensetzung eine maximale Kraft von 30 N benötigt, um durch die Kanüle hindurch zu gelangen.

## Revendications

1. Composition viscoélastique comprenant une solution aqueuse contenant au minimum environ 0,0 1 % p/v et au maximum environ 20% p/v d'un polymère viscoélastique par rapport au volume total de la composition viscoélastique et comprenant en outre du tris[hydroxyméthyl]aminométhane.

2. Composition selon la revendication 1, dans laquelle la concentration en tris[hydroxyméthyl]aminométhane est au maximum d'environ 50 mM et au minimum d'environ 0,1 mM par rapport au poids total de la composition viscoélastique.

3. Composition selon la revendication 1, comprenant en outre un polyol.

4. Composition selon la revendication 3, dans laquelle au moins un polyol est choisi dans le groupe constitué par les pentanols, les hexanols et les heptanols et les mélanges de ceux-ci.

5. Composition selon la revendication 4, dans laquelle le polyol est un hexanol.

6. Composition selon la revendication 5, dans laquelle le polyol est le mannitol.

7. Composition selon la revendication 5, dans laquelle le polyol est le sorbitol.

8. Composition selon la revendication 3, dans laquelle la concentration en polyol est au minimum d'environ 0, 1 % p/v et au maximum d'environ 15% p/v par rapport au volume total de la composition viscoélastique.

9. Composition selon la revendication 1, dans laquelle la concentration en tris[hydroxyméthyl]aminométhane est au minimum d'environ 0,5 mM et au maximum d'environ 30 mM.

10. Composition selon la revendication 1, dans laquelle le rapport de la viscosité de la composition viscoélastique à la viscosité d'une composition viscoélastique comparable ne contenant ni polyol ni tris[hydroxyméthyl]aminométhane est au minimum d'environ 1 et au maximum d'environ 2,5.

11. Composition selon la revendication 1, dans laquelle le pourcentage d'affaiblissement est au minimum d'environ 45%.

12. Composition selon la revendication 1, dans laquelle le polymère viscoélastique est choisi dans le groupe constitué par l'acide hyaluronique, l'hydroxypropylméthylcellulose, le poly(acide acrylique), le carbopol, le poly(alcool vinylique), la polyvinylpyrrolidone, le sulfate de chondroïtine, le polycarbophile, la méthylcellulose, la carboxy-méthylcellulose, l'hydroxyéthylcellulose, l'hydroxypropylcellulose, l'éthylcellulose, les poly(oxyde d'éthylène), l'alginate, la pectine, la gomme xanthane, les dextranes, le collagène et leurs dérivés et leurs sels et leurs combinaisons.

13. Composition selon la revendication 1, dans laquelle le polymère viscoélastique comprend un polysaccharide.

14. Composition selon la revendication 13, dans laquelle le polysaccharide est choisi dans le groupe constitué par l'acide hyaluronique, l'hydroxypropylméthylcellulose, le sulfate de chondroïtine, la méthylcellulose, la carboxyméthylcellulose, l'hydroxyéthylcellulose, l'hydroxypropylcellulose, l'éthylcellulose, l'alginate, la pectine, les dextranes, le collagène, les protéoglycanes, la polyvinylpyrrolidone, la kératine, les carraghénines et leurs dérivés et leurs sels et leurs combinaisons.

15. Composition selon la revendication 1, dans laquelle le polymère viscoélastique comprend un alginate.

16. Composition selon la revendication 15, dans laquelle la concentration de l'alginate est au minimum d'environ 0,05% p/v et au maximum d'environ 9% p/v par rapport au volume total de la composition viscoélastique.

17. Composition selon la revendication 15, dans laquelle la masse moléculaire moyenne de la composition d'alginate va d'environ 50 kD au minimum à environ 5 000 kD au maximum.

18. Composition selon la revendication 1, dans laquelle le polymère viscoélastique comprend un mélange d'acide hyaluronique et/ou de ses sels et d'hydroxypropylméthylcellulose.

19. Composition selon la revendication 18, dans laquelle la concentration de l'acide hyaluronique et/ou de ses sels est au minimum d'environ 0, 1 % p/v et au maximum d'environ 6% p/v par rapport au volume total de la composition viscoélastique.

20. Composition selon la revendication 19, dans laquelle la masse moléculaire moyenne de la composition d'acide hyaluronique et/ou de ses sels va d'environ 500 kD au minimum à environ 5 000 kD au maximum.

21. Composition selon la revendication 18, dans laquelle la concentration en hydroxypropylméthylcellulose est au minimum d'environ 0,05% p/v et au maximum d'environ 5% p/v par rapport au volume total de la composition viscoélastique.

22. Composition selon la revendication 21, dans laquelle la masse moléculaire moyenne de l'hydroxypropylméthylcellulose va d'environ 10 kD au minimum à environ 120 kD au maximum.

23. Composition selon la revendication 1, dans laquelle la masse moléculaire moyenne du polymère viscoélastique va d'environ 20 kD au minimum à environ 5 000 kD au maximum.

24. Composition selon la revendication 1, dans laquelle l'osmolalité de la composition viscoélastique va d'environ 200 mOsmol/kg au minimum à environ 400 mOsmol/kg au maximum.

25. Composition selon la revendication 1, dans laquelle la viscosité à un taux de cisaillement nul de la composition viscoélastique va d'environ 6.10⁴ cps au minimum à environ 4.10⁶ cps au maximum.

26. Composition selon la revendication 1, dans laquelle la viscosité à un taux de cisaillement élevé de la composition viscoélastique va d'environ 500 cps au minimum à environ 2 000 cps au maximum.

27. Composition selon la revendication 1, dans laquelle le pH de la composition viscoélastique est au minimum d'environ 5 et au maximum d'environ 8.

28. Trousse pour une composition viscoélastique, la trousse comprenant une seringue contenant la composition viscoélastique selon la revendication 1.

29. Trousse selon la revendication 28, dans laquelle la seringue possède un orifice de sortie, la trousse comprend en outre une canule conçue pour être connectée de manière étanche à l'orifice de sortie ayant un diamètre intérieur maximal d'environ 2 mm.

30. Trousse selon la revendication 28, dans laquelle la composition viscoélastique nécessite une force maximale de 30 N pour traverser la canule.
